# EUROPEAN PATENT SPECIFICATION

(11) **EP 2 024 763 B1**
(45) Date of publication and mention of the grant of the patent: **02.09.2015**
(21) Application number: 07712832.0
(22) Date of filing: 06.03.2007
(51) Int. Cl.: G01V 9/00

(54) **METHODS AND SYSTEMS FOR EVALUATION OF HYDROCARBON RESERVOIRS AND ASSOCIATED FLUIDS USING BIOLOGICAL TAGS AND REAL-TIME PCR**
METHODEN UND SYSTEME ZUR EVALUIERUNG VON ÖLVORKOMMEN UND MIT DIESEN ASSOZIIERTEN FLÜSSIGKEITEN UNTER VERWENDUNG BIOLOGISCHER MARKER UND ECHT-ZEIT PCR
PROCÉDÉS ET SYSTÈMES POUR L'ÉVALUATION DE RÉSERVOIRS D'HYDROCARBURES ET FLUIDES ASSOCIÉS UTILISANT DES MARQUEURS BIOLOGIQUES ET UNE PCR EN TEMPS RÉEL

(30) Priority: 17.05.2006 GB 0609735
(43) Date of publication of application: 18.02.2009
(73) Proprietor: Schlumberger Technology B.V., 2514 JG The Hague (NL); Schlumberger Holdings Limited, Tortola (CA); Services Pétroliers Schlumberger, 75007 Paris (FR); PRAD Research and Development N.V., Willemstad, Curacao (AN)
(72) Inventor: PELHAM, Sarah, Cambridge CB1 1HR (GB); TUSTIN, Gary, Sawston, Cambridgeshire CB2 4DQ (GB); BARROW, Harry, Girton, Cambridgeshire CB3 0QW (GB)
(74) Representative: Li, Boxi
(86) International application number: PCT/GB2007/000762
(87) International publication number: WO 2007/132137

(56) References cited:
- WO-A-01/81914
- WO-A2-2004/022785
- ORPHAN V J ET AL: "Culture-dependent and culture-independent characterization of microbial assemblages associated with high-temperature petroleum reservoirs" APPLIED AND ENVIRONMENTAL MICROBIOLOGY, WASHINGTON,DC, US, vol. 66, no. 2, February 2000 (2000-02), pages 700-711, XP002273778 ISSN: 0099-2240
- BELLER H.R., STACI R. KANE, LEGLER T., ALVAREZ P.J.J.: "A Real Time Polymerase Chain Reaction Method for Monitoring Anaerobic, Hydrocarbon-Degrading Bacteria Based on a Catabolic Gene", ENVIRONMENTAL SCIENCE AND TECHNOLOGY, vol. 36, no. 18, 8 October 2002 (2002-10-08), pages 3977-3984,

## Description

### BACKGROUND OF THE INVENTION

Embodiments of the present invention relate to characterization of hydrocarbon reservoirs and/or determining flow properties of fluids associated with such reservoirs. More specifically, but not by way of limitation, embodiments of the present invention provide for using biological tags as tracers that may be detected and/or measured in fluids retrieved from the hydrocarbon reservoir, one or more wellbores associated with the hydrocarbon reservoir or one or more locations proximal to the hydrocarbon reservoir using a real-time polymerase chain reaction ("real-time PCR"). From the detection and/or measurement of the biological tags in the retrieved fluids, the reservoir may be characterized and/or flow properties of fluids associated with the hydrocarbon reservoir may be determined. The fluids associated with the hydrocarbon reservoir may be fluids existing in the hydrocarbon reservoir and/or adjacent earth formations or may be fluids introduced into the hydrocarbon reservoir.

In the specification, the term "real-time PCR" may refer to the monitoring of the amplification process in a polymerase chain reaction as the polymerase chain reaction proceeds. In certain aspects, the monitoring of the polymerase chain reaction may be provided by monitoring amplicon produced in the polymerase chain reaction, and such monitoring may be performed during one or more of the cycles of the polymerase chain reaction.

Previously, characterization of reservoirs in earth formations or flow properties of fluids through earth formations has been provided for by the use of radioactive or chemical tracers. Traditional radioactive tracers include: the radionuclides 57Co, 58Co and 60Co (that may be incorporated into anionic complexes, such as hexacyanocobaltates *etc*.); 134Cs, 137Cs (as a corresponding chloride); tritiated water; and 22Na as sodium chloride. The most common beta emitting radioactive tracers for inter-well studies may be labelled with tritium (tritiated water) and carbon-14 (labelled thiocyanate). In radioactive tracer techniques, liquid scintillation counting may be used to detect the tracers found in fluid samples taken from earth formations or proximal locations.

With regard to chemical tracers, some of the chemicals that have been used for reservoir characterization include chloride, bromide, iodide, nitrate, thiocyanate, fluorescein, rhodamine, 2-propanol, t-butanol and other water-soluble small organic molecules. In such techniques, after sample collection, detection of the chemicals may be conducted by techniques such as ion chromatography (for multiple anions), high-performance liquid chromatography, ultraviolet spectroscopy (for thiocyanate), colorimetry/fluorimetry (for dyes), conductivity (for ionic species), gas-liquid chromatography (for small organics). With chemical tracing, the detection limit - along with an estimate of the reservoir pore volume and expected dilution - determines the overall quantity of the chemical to be introduced into the hydrocarbon reservoir. Since a better description of the reservoir properties is the object of a tracer study, there may be a need to use large amounts of chemicals, which may be expensive and raise ecological/waste management issues. As such, the amount of tracer used is a balance between ensuring a measurable signal at the monitoring point(s) but with reasonable cost.

Both radioactive and chemical tracer techniques, in practice, have many limitations, including that radioactive tracers require specialized environmental measures/precautions and chemical tracers require application of large doses of chemicals to provide for accurate detection and quantitative analysis. Moreover, with both radioactive and chemical tracers separate detection tests needs to be performed when more than one type of radioactive or chemical tracer is applied to the hydrocarbon reservoir. Furthermore, with regard to oil and gas reservoirs, processes such as hydraulic fracturing, hydrocarbon recovery processes and the like may have several stages and each stage of the process may have a fluid associated with the stage, including in fracturing, for example, pre-pad fluids, pad fluids, fracturing fluids and tailing fluids. Although these procedures may now be established in the oil and gas industry, there still exists significant room for improvement to the processes that may require understanding movements of the different fluids through the hydrocarbon reservoir and adjacent formations.

WO 01/81914 discloses a method for monitoring the hydrocarbon and water production from different production zones in a hydrocarbon reservoir and/or injection wells and detection of different phenomena. The method comprises dividing regions around wells in the reservoir into a number of sections, and injecting or placing specific tracers with unique characteristics for each section into the formation in these regions. The tracers may be chemically intelligent and released as a function of specific events.

### BRIEF SUMMARY OF THE INVENTION

Embodiments of the present invention provide methods and systems for characterizing a hydrocarbon reservoir and/or determining flow characteristics of one or more liquids injected into and/or flowing in the hydrocarbon reservoir using biological tags and a real-time polymerase chain reaction. In one embodiment of the present invention, characterization of a hydrocarbon reservoir or determining flow properties of fluids associated with the hydrocarbon reservoir may be provided by adding a biological tag to a liquid injected into and/or associated with the hydrocarbon reservoir, collecting a fluid sample from the hydrocarbon reservoir, performing real-time PCR on the fluid sample to detect a presence of and/or measure an amount of the biological tag in the fluid sample, and using results from the real-time PCR to provide for at least one of the characterization of the hydrocarbon reservoir and determining flow properties of the liquid injected into and/or associated with the hydrocarbon reservoir.

More specifically, but not by way of limitation, in embodiments of the present invention: (a) biological tags may be added to one or more liquids associated with the hydrocarbon reservoir, such as liquids injected into, liquids found in or flowing through the reservoir; (b) samples may be collected from the hydrocarbon reservoir or surrounding locations and multiple samples may be taken at different geographical or temporal locations; (c) the presence of the biological tag may be qualitatively and/or quantitatively tested for in the samples using real-time PCR; and (d) from the qualitative and/or quantitative results of the real-time PCR analysis the hydrocarbon reservoir may be characterized, the flow of the one or more liquids in the hydrocarbon reservoir may be mapped and/or where the one or more liquids has been added to the hydrocarbon reservoir - for a purpose such as fracturing, hydrocarbon recovery or the like - an analysis of the likely results of the fracturing process, hydrocarbon recovery or the like may be determined..

In one embodiment, a hydrocarbon reservoir is characterized and/or flow properties of fluids in the hydrocarbon reservoir may be determined by injecting a first liquid containing a first biological tag into the hydrocarbon reservoir or adding the first biological tag to a liquid associated with the hydrocarbon reservoir, collecting a first fluid sample from the hydrocarbon reservoir, performing real-time PCR on the first fluid sample using a first pair of complementary primers in a polymerase chain reaction ("PCR"), wherein the first pair of complementary primers are configured to anneal to single strands of the first biological tag and provide for replication of the first biological tag during the real-time PCR, and wherein the step of performing real-time PCR comprises detecting PCR product produced by the replication of the first biological tag in the PCR reaction and using results from the real-time PCR to provide for at least one of characterization of the hydrocarbon reservoir and determining flow of the first liquid in the hydrocarbon reservoir.

In certain aspects of the above embodiment, the biological tag may be DNA sequences and the first pair of complementary primers may be DNA fragments having DNA sequences complementary to the ends of the DNA sequence of the biological tag. In an embodiment of the present invention, the real-time PCR process may involve the use of probes configured to provide a detectable physical property that varies in accordance with amount of the PCR product, amplicon, produced during the real-time PCR. In such an embodiment, a value of the physical property may be measured to determine the presence of the PCR product and/or a relative quantity of the PCR product present. The PCR product may be the biological tags originally present in the sample undergoing real-time PCR, copies of the original biological tags generated by the PCR process and incomplete copies of the biological tags being generated in the PCR - *i.e.,* the denatured single strands of the biological tags combined with the primers and/or nucleotide bases that are in the process of forming molecules equivalent to the biological tags.

In certain aspects of embodiments of the present invention, the biological tags may be introduced into the hydrocarbon reservoir via a wellbore. In such aspects, the biological tags may be introduced into the hydrocarbon reservoir in a fracturing fluid, injection water, drilling fluid, tracer fluid and/or the like. Coiled tubing drilling techniques may be used in some aspects to provide for delivery of the biological tags into the hydrocarbon reservoir, a wellbore in the hydrocarbon reservoir, an earth formation proximal to the hydrocarbon reservoir and/or the like.

In another embodiment of the present invention, a method for characterizing a hydrocarbon reservoir and determining flow of a plurality of fluids in the hydrocarbon reservoir is provided that comprises:
injecting a first liquid containing first biological tags into the hydrocarbon reservoir;
injecting a second liquid containing second biological tags into the hydrocarbon reservoir;
collecting a first fluid sample from the hydrocarbon reservoir;
performing real-time PCR on the first fluid sample using a real-time PCR mixture, wherein the real-time PCR comprises:
   using a first primer to amplify any of the first biological tags present in the first fluid sample, wherein the first primer is configured to selectively attach to the first biological tags and provide for amplification of the first biological tags;
   using a second primer to amplify any of the second biological tags present in the first fluid sample, wherein the second primer is configured to selectively attach to the second biological tags and provide for amplification of the second biological tags;
   using a first probe to selectively detect the presence of the first biological tags, wherein the first probe is configured to produce a first measurable physical property that varies in accordance with a first amount of the first biological tags present in the real-time PCR mixture;
   using a second probe to selectively detect the presence of the second biological tags, wherein the second probe is configured to produce a second measurable physical property that is distinct from the first measurable physical property and that varies in accordance with a second amount of the second biological tags present in the real-time PCR mixture; and
using results from the real-time PCR to determine flow properties of the first and the second fluids in the hydrocarbon reservoir.

In such an embodiment, the first and second liquids may be injected into the hydrocarbon reservoir through a borehole penetrating the hydrocarbon reservoir.

In a further embodiment of the present invention, a system for characterizing a hydrocarbon reservoir and determining flow of fluids in the hydrocarbon reservoir is provided that may comprise a first well-tool configured to pump a first liquid containing a biological tag into the hydrocarbon reservoir, a sampling chamber configured to collect a fluid sample from the hydrocarbon reservoir, a real-time PCR device configured to perform real-time PCR of the fluid sample and to detect/measure the biological tag, and a processor configured to process outputs from the real-time PCR device to determine at least one of characterization of the hydrocarbon reservoir and flow of the first liquid in the hydrocarbon reservoir.

Reference to the remaining portions of the specification, including the drawings and claims, will realize other features and advantages of the present invention. Further features and advantages of the present invention, as well as the structure and operation of various embodiments of the present invention, are described in detail below with respect to the accompanying drawings.

### BRIEF DESCRIPTION OF THE DRAWINGS

The present invention will become more fully understood from the detailed description and the accompanying drawings, wherein:
Fig. 1A is a schematic-type illustration of a wellbore penetrating a hydrocarbon reservoir and an associated wellbore assembly for introducing fluids containing biological tags into a hydrocarbon reservoir, in accordance with an embodiment of the present invention;
Fig. 1B is a schematic-type illustration of a sampling well-tool suspended in a wellbore penetrating a hydrocarbon reservoir configured for sampling fluids associated with the hydrocarbon reservoir and an associated real-time polymerase chain reaction processing station, in accordance with an embodiment of the present invention:
Fig. 2A is a flow type schematic illustrating a conventional polymerase chain reaction process;
Fig. 2B is a flow type schematic illustrating a real-time polymerase chain reaction process for analyzing samples of fluids associated with a hydrocarbon reservoir, in accordance with an embodiment of the present invention; and
Fig. 3 is a flow type schematic illustrating a method for characterizing a hydrocarbon reservoir and/or determining flow properties of one or more liquids associated with the hydrocarbon reservoir, in accordance with an embodiment of the present invention.

In the appended figures, similar components and/or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.

### DETAILED DESCRIPTION OF THE INVENTION

Specific details are given in the following description to provide a thorough understanding of the embodiments. However, it will be understood by one of ordinary skill in the art that the embodiments may be practiced without these specific details. For example, circuits may be shown in block diagrams in order not to obscure the embodiments in unnecessary detail. In other instances, well-known circuits, processes, algorithms, structures, and techniques may be shown without unnecessary detail in order to avoid obscuring the embodiments.

Also, it is noted that the embodiments may be described as a process which is depicted as a flowchart, a flow diagram, a data flow diagram, a structure diagram, or a block diagram. Although a flowchart may describe the operations as a sequential process, many of the operations can be performed in parallel or concurrently. In addition, the order of the operations may be re-arranged. A process is terminated when its operations are completed, but could have additional steps not included in the figure. A process may correspond to a method, a function, a procedure, a subroutine, a subprogram, etc. When a process corresponds to a function, its termination corresponds to a return of the function to the calling function or the main function.

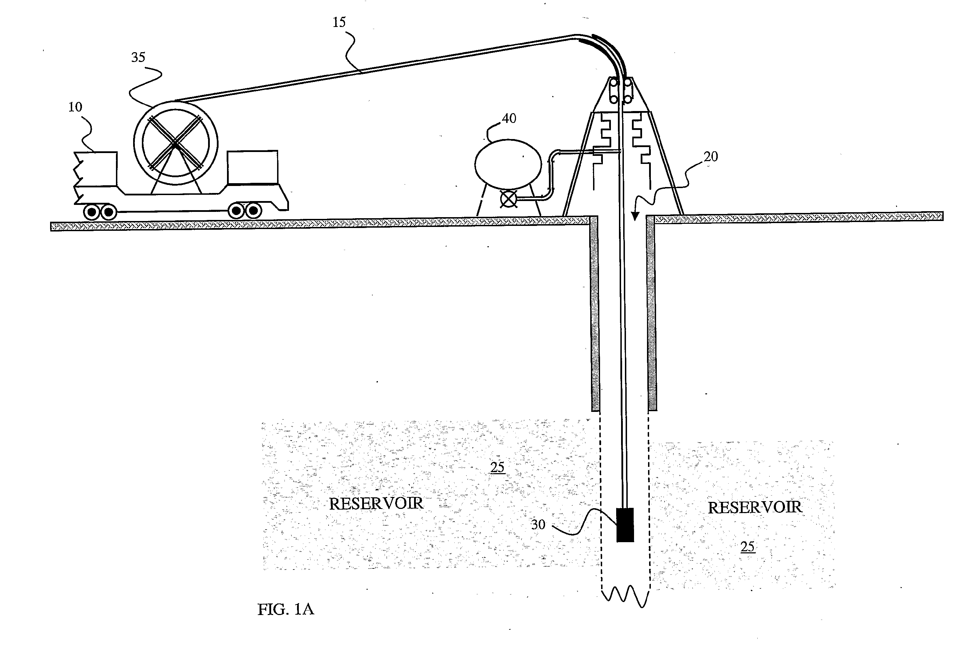

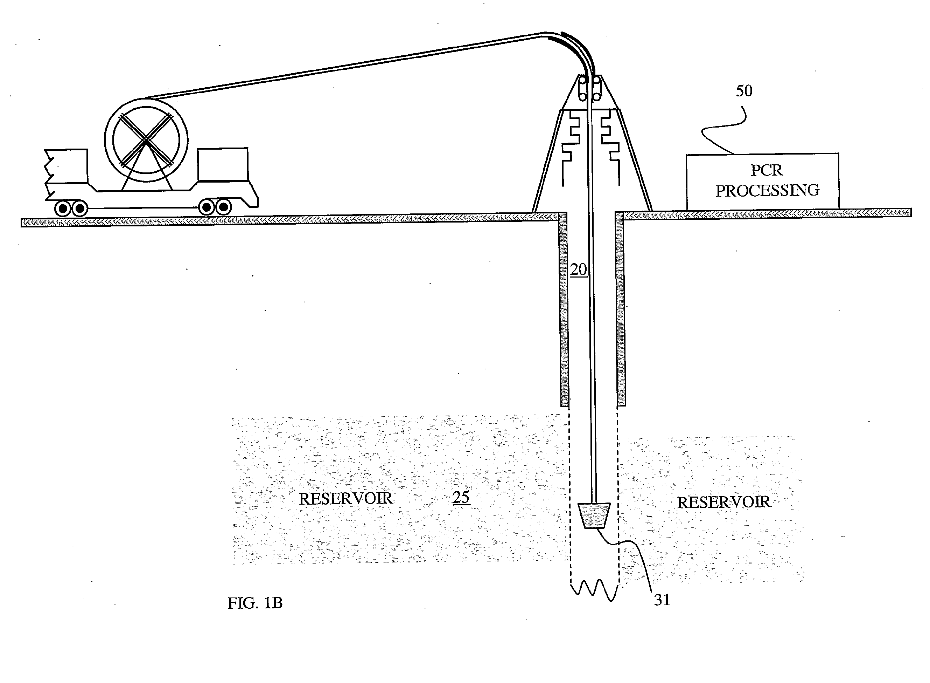

Fig. 1A is a schematic-type illustration of a wellbore penetrating a hydrocarbon reservoir and an associated wellbore assembly for introducing fluids into a hydrocarbon reservoir, in accordance with an embodiment of the present invention. Referring now to Fig. 1, a truck winch 10 or the like may be used to wind or unwind a wireline 15 or the like into and out of a wellbore 20 that penetrates a hydrocarbon reservoir 25. The wireline 15 may be coupled with a well-tool 30 and provide for the movement of the well-tool 30 in the wellbore 20, which may include movement of the well-tool 30 to locations within the hydrocarbon reservoir 25. Positioning of the well-tool 30 in the wellbore 20 may be provided by a positioning wheel 35 or the like, which may be configured to maneuver the well-tool 30 in the wellbore 20.

In some embodiments of the present invention a pump 40 or the like may be used to introduce fluids into the hydrocarbon reservoir 25. Fluids may be pumped directly into the wellbore 20 or may be introduced into the hydrocarbon reservoir 25 by the well-tool 30, which may be coupled with the pump 40. As persons of skill in the art are aware, perforations may be made in the casing of the wellbore 20 to provide for the application of fluids to different formation locations adjacent to the wellbore 20. Further, methods of sealing off parts and/or sections of the wellbore 20 and plugging the perforations after introduction of fluids may provide for more precise, forceful and/or economical delivery of fluids to formations adjacent to the wellbore 20. Using the described methods of entering a fluid into the hydrocarbon reservoir, in an embodiment of the present invention, a fluid containing biological tags may be introduced into the hydrocarbon reservoir.

In certain aspects, coiled tubing techniques may be used as a means to provide for application/retrieval of a fluid containing the biological tags into/from the hydrocarbon reservoir 25 and/or an adjacent location. Coiled tubing is a long, continuous string of tubing, generally made of steel that may be used in the drilling of a borehole. Coiled tubing is increasing in popularity as a method of conducting operations in an oil or gas wellbore. Historically, drilling pipe was used for drilling and conducting operations inside a wellbore, usually several hundred or thousand feet under the surface of the ground. However, drill pipe must be assembled in sections and lowered into the wellbore over a long time period of many hours or days. Coiled tubing has emerged as one solution to this matter by providing a relatively fast and reliable method of conducting operations downhole within a wellbore, without using heavy and cumbersome jointed drilling pipe. Coiled tubing is used as a continuous strand, and therefore is easier and faster to use in many wellbore operations. Technological developments, improved service reliability, and the need to drive down industry costs have contributed to expanded uses for coiled tubing.

In certain embodiments, a coiled tube borehole may be provided adjacent to and/or within another borehole associated with the hydrocarbon reservoir 25 and/or into or adjacent to the hydrocarbon reservoir 25. As such, a borehole created by a coiled tubing drilling technique may be used to provide for introduction/injection of the biological tags to desired locations associated with the hydrocarbon reservoir 25 and/or sampling of fluids at locations associated with the hydrocarbon reservoir 25.

The fluid containing the biological tags and being added to and/or injected into the hydrocarbon formation may be a treatment fluid - *i.e*., a fluid configured to provide for such things as stimulation, isolation or control of reservoir gas or water - a fluid for use in a hydraulic fracturing process - such as a pre-pad fluid, a pad fluid, a fracturing fluid, a tailing fluid or the like - and/or the like. Alternatively or in combination, in certain embodiments of the present invention, biological tags may be added to a fluid already in or flowing through the hydrocarbon reservoir to determine flow properties for the fluid, determine changes to the fluids flow during extraction of hydrocarbons from the reservoir and/or the like. Such fluids may include formation liquid hydrocarbons, water and/or the like.

Fig. 1B is a schematic-type illustration of a sampling well-tool suspended in a wellbore penetrating a hydrocarbon reservoir configured for sampling fluids associated with the hydrocarbon reservoir and an associated real-time PCR processing station, in accordance with an embodiment of the present invention. As illustrated, in an aspect of the present invention a sampling tool 31 may be entered into the hydrocarbon reservoir 25 via the wellbore 20. In such an aspect, the sampling tool 31 may be used to collect fluid samples from inside the wellbore 20. In other aspects, fluid samples may be collected from locations including one or more of the following or the like: locations adjacent to the hydrocarbon reservoir 25 that may be inside or outside an earth formation; different locations inside the wellbore 20; a sampling borehole penetrating the hydrocarbon reservoir 25, a sampling borehole penetrating an earth formation outside of the reservoir 25. In some embodiments of the present invention, fluid samples may be collected at different geographical and or temporal locations. In such embodiments, the plurality of samples may be analyzed using real-time PCR and the results may be processed along with geographical and temporal data to provide for fluid flow analysis and/or reservoir characterization.

In an embodiment of the present invention, the collected samples of the fluid may be taken to a real-time PCR processing station 50. The real-time PCR processing station 50 may be at the wellhead, an onsite laboratory, an offsite laboratory or the like. The real-time PCR processing station may contain a real-time PCR processor, related computer systems, fluid analyzers and/or the like. At the real-time PCR processing station 50, real-time PCR analysis of the collected samples may be performed to determine fluid flow type properties of fluids introduced into the wellbore 20, associated with the hydrocarbon reservoir 25, associated with adjacent earth formations (such as formation water) and/or the like. The processing station 50 may be networked with one or more pieces of equipment associated with the wellhead, wellbore and/or the reservoir and may provide for adjusting controls of the one or more pieces of equipment in accordance with results obtained from the real-time PCR analysis of the one or more of the fluid samples.

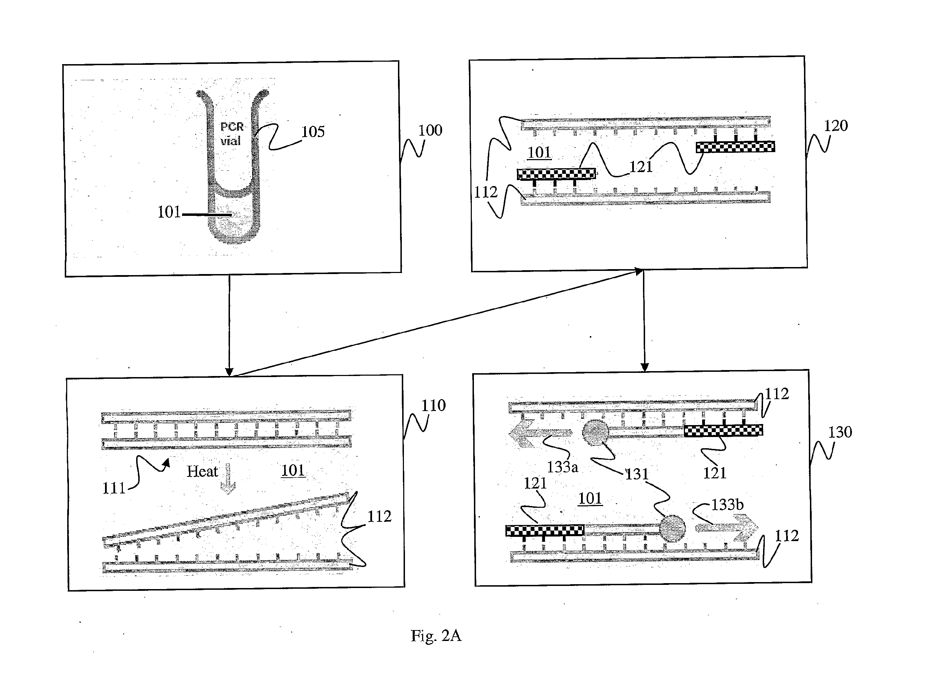

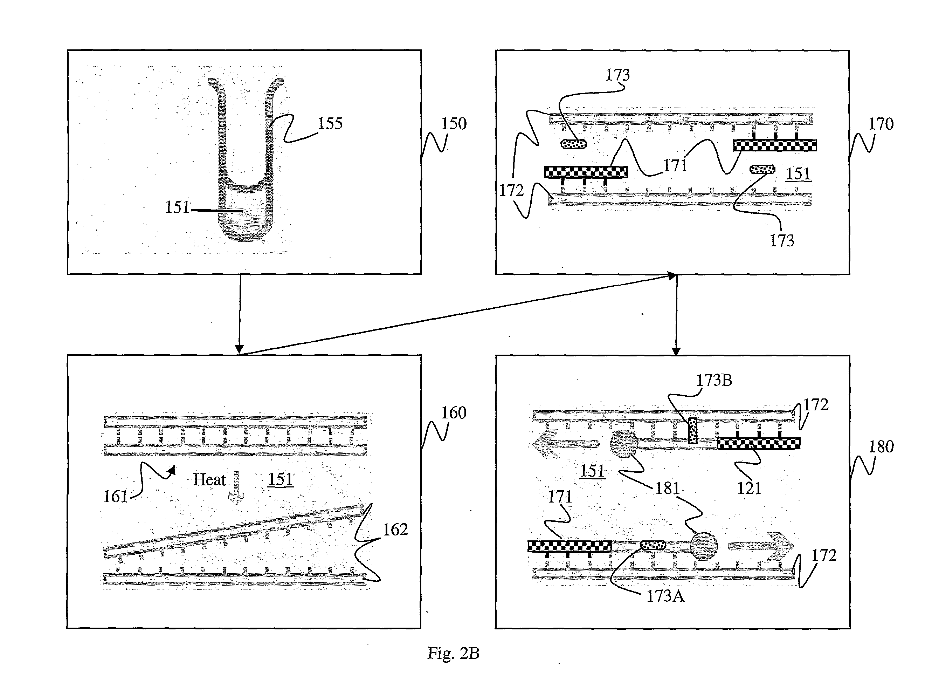

Fig. 2 illustrates a conventional PCR process. In step 100 of the conventional PCR process, reactants may be mixed together as a PCR reaction mixture 101 in a PCR test tube 105, which may be a test tube, vial, reaction vessel and/or the like. The PCR reaction mixture 101 of the conventional process may comprise the DNA to be amplified (in embodiments of the the present invention this may be the biological tags or equivalents), a DNA polymerase enzyme, small primer sequences of DNA, a supply of nucleotide bases and/or magnesium chloride.

In step 110 of the conventional PCR process, the PCR reaction mixture 101 may be heated. This heating may occur in a specialized PCR machine designed to provide for performing the conventional PCR process. In certain aspects, the reaction mixture 101 may be heated to between 90-100 degrees Celsius or the like and this heating may take place for periods in the region of thirty seconds. At temperatures in excess of about 94 degrees Celsius, complete DNA strands 111 present in the PCR reaction mixture 101 may separate into single DNA strands 112 as the hydrogen bonds holding them together break down.

In step 120 of the conventional PCR process, the PCR reaction mixture 101 containing the separated DNA strands 112 mixture may be cooled down. Cooling of the temperature of the reaction mixture 101 in this step may be to temperatures in the range of about 50 to 60 degrees Celsius. At such a temperature range, primers 121 contained in the reaction may bind/anneal to the single DNA strands 112. The primers 121 may comprise short sequences of nucleotide bases which join to the beginning of the single DNA strands 112 to provide for the copying/amplification process to start. The primers 121 may join to the beginning of the separated DNA strands to provide for the full copying process to start. Busing complementary pairs of primers, it may be provided that the areas on either side of the target sequence are extended.

The primers 121 have DNA sequences complementary to areas adjacent to each side of the target sequence. As such, if the DNA sequence around the region selected to be amplified is known, the correct primers may be chosen to provide for amplification of only the selected DNA sequence or maybe very close variants. In embodiments of the present invention, because the DNA sequences of the biological tags applied to the hydrocarbon reservoir are known/chosen, primers may be selected that will only provide for amplification of DNA fragments with the same DNA sequence as the biological tags and the biological tags may be selected with DNA sequences that are unlikely to occur "naturally" in the geological formation, hydrocarbon reservoir or fluid being analyzed. As such, embodiments of the present invention may be very accurate in detecting small amounts of the biological tags being used as tracers with a very low risk of contamination from DNA fragments naturally occurring in the hydrocarbon reservoir. The primers 121 may be constructed in a laboratory or purchased from commercial suppliers.

In step 130 of the conventional PCR process, the PCR reaction mixture 101 may be heated up to a temperature in the range of about 70 to 80 degrees Celsius. At this temperature, DNA polymerase enzyme 131 may act to add bases to the primers 121 so as to build up, as illustrated by arrows 133a and 133b, strands of DNA complementary to the single DNA strands 112 so as to form complete molecules/DNA fragments that are identical to the molecules/DNA fragments of the original DNA molecules/fragments that were provided in the PCR test tube 105 in step 100 to be amplified/copied. Steps 110, 120 and 130 may be repeated in what are known as cycles to provide for further amplification/copying of the selected DNA. By repeating steps 110, 120 and 130 around thirty times, it is possible to produce in the order of 1 billion copies of the original DNA selected for amplification/copying. However, such processing may take in the order of several hours, and, in conventional PCR is only a qualitative process, not quantitative, in that it only establishes the presence of a certain DNA in the PCR reaction mixture 101, not how much of the certain DNA was originally present in the PCR test tube 105.

Fig. 2B is a flow type schematic illustrating a real-time PCR process for analyzing fluids associated with a hydrocarbon recovery process, in accordance with an embodiment of the present invention. In an embodiment of the present invention, as discussed in more detail previously, biological tags may be introduced into a fluid associated with a hydrocarbon reservoir to act as tracers. Fluid samples may then be sampled from the reservoir or areas proximal to the reservoir to test for the biological tags and, as a result, to determine flow properties of the fluid and/or characterization of the hydrocarbon reservoir.

In an embodiment of the present invention, in step 150, a sample - the sample having been taken from the hydrocarbon reservoir, a wellbore associated with the reservoir or a location associated with the hydrocarbon reservoir after introduction of biological tags into one or more fluids associated with the hydrocarbon reservoir - is placed in a test tube 105 (which may be a vial, chemical container or the like) and mixed with a real-time PCR reaction mixture 151 comprising a DNA polymerase enzyme, primer sequences of DNA, a supply of nucleotide bases, some real-time PCR probes and/or the like.

As discussed above, the biological tags and the primers may be selected so as to be complimentary to provide for selective copying/amplification of only any DNA fragments in the fluid sample that have the same DNA sequence as the biological tags. This use of primers specific to the biological tags introduced into the fluid associated with the hydrocarbon reservoir may provide for accurately detecting low levels of the DNA fragments because, among other reasons, there is little background noise from detections of similar DNA fragments and probabilities that DNA fragments matching the DNA fragments introduced into the fluid may occur in the sample from a source other than the deliberate introduction for flow analysis, as described above, are very low.

The real-time PCR probes in the real-time PCR reaction mixture 151 may comprise binding dyes - such as SYBR^{®} Green - hybridization or hydrolysis probes (that may be fluorescently labeled sequence-specific probes) such as QuantiProbes^{®}, TaqMan^{®} probes, FRET probes, molecular beacons and/or the like. Further, in certain aspects, the real-time PCR reaction mixture 151 may comprise primers/probes such as Scorpion^{™} probes, Sunrise^{™} primers, LUX^{™} fluourogenic primers and/or the like.

In step 160, the real-time PCR reaction mixture 151 may be heated. In certain aspects the real-time PCR reaction mixture 151 may be heated to a temperature above about 94 degree Celsius or the like to provide for denaturing of any DNA fragments 161 in the real-time PCR reaction mixture 151 into single DNA fragment strands 162 as the hydrogen bonds holding the DNA strands together break down.

In step 170, the real-time PCR reaction mixture 151 containing the single DNA fragment strands 172 may be cooled down. Cooling of the temperature of the real-time PCR reaction mixture 151 in this step may be to temperatures in the range of about 50 to 60 degrees Celsius. At such a temperature range, any primers in the reaction may bind/anneal to any single DNA fragment strands. In an embodiment of the present invention, primers 171 may comprise short sequences of nucleotide bases which may be specifically selected to join to an end of single tracer DNA fragment strands 172 (where the single tracer DNA fragment strands 172 are formed from the denaturing in step 160 of the biological tags) to provide for the copying/amplification of the single tracer DNA fragment strands 172. In this way the primers 171 may provide for selective amplification/copying of only the single tracer DNA fragment strands 172, and in turn the selective amplification of the biological tags in the sample.

In certain embodiments of the present invention, more then one biological tag may be introduced into the hydrocarbon reservoir or fluids associated with the hydrocarbon reservoir. For example, multiple liquids may be used in a fracturing process performed on the hydrocarbon reservoir and/or surrounding earth formations and each liquid may be provided with its own unique biological tag. As such, the primers 171 may comprise more than one type of primer, where each type of primer may be selected to provide for amplification of any of the different biological tags being used as tracers in the reservoir. In alternative embodiments, the primers 171 may be selected so that the primers 171 provide for joining with all of the different biological tags used. In this way, the same primers, the primers 171, may provide for the amplification of all of the different biological tags.

In an embodiment of the present invention, the real-time PCR reaction mixture 151 may contain probes 173. In some embodiments of the present invention, the probes 173 may not be separate elements, but, rather, may be incorporated into and/or associated with the primers 171 and/or other elements/compounds in the real-time PCR reaction mixture 151. The probes may be configured to provide/create/cause/be the source of a measurable physical effect that changes with the amount of PCR product in the real-time PCR reaction mixture 151; where the PCR product, in some embodiments of the present invention, is the DNA product produced during the amplification/copying of the biological tags and may include the original biological tags and copies of the biological tags produced during the PCR process. In other embodiments of the present invention, the probes 173 may be responsive to incomplete copies of the biological tags that may comprise the single tracer DNA fragment strands 172 - the single tracer DNA fragment strands 172 resulting from denaturing of the biological tags or in subsequent cycles resulting from the denaturing of the copies of the biological tags - coupled with primers and/or nucleotide bases. As such, in embodiments of the present invention the probes 173 are selected to produce a measurable physical effect during the amplification of the biological tags and the measurable physical effect provided is chosen so that it varies in accordance with the amount of the PCR product present in the real-time PCR mixture 151. In aspects in which multiple biological tags are introduced into the hydrocarbon reservoir and the primers 171 are selected to provide for amplification of all of the different biological tags, the probes 173 may be selected so that the amount of amplicon produced from the amplification of the different biological tags may be analyzed, determined and/or measured.

In step 180, the real-time PCR reaction mixture 151 may be heated up to a temperature in the range of about 70 to 80 degrees Celsius. At this temperature, DNA polymerase enzyme 181 may act to add bases to the primers 171 so as to build up complementary strands of DNA that are identical to the single tracer DNA fragment strands 172 to produce molecules equivalent to the biological tags. In this way, the biological tags, if present in the sample, may be amplified/copied.

The probes 173 are configured to react with the primers 171 and/or the single tracer DNA fragment strands 172 as the primers 171 and the single tracer DNA fragment strands 172 interact to produce copies of the biological tags, if present. This reaction of the probes 173 with the primers 171 and/or the single tracer DNA fragment strands 172 may occur in step 170 (not shown) and/or step 180. Merely by way of example, a probe such as SYBR® Green fluoresces in the presence of double stranded DNA so as to produce a measurable physical effect, fluorescence, in the presence of the biological tags and copies of the biological tags. Because SYBR® Green may produces fluorescence in the presence of any double stranded DNA, it may not be used as a selective probe. A quantiprobe on the other hand may be sequence specific and may produce fluorescence during the annealing process of step 170.

Steps 110, 120 and 130 may be repeated to provide for further amplification/copying of the selected DNA. By repeating the steps around thirty times or the like, it may be possible to produce in the order of 1 billion copies of the original DNA selected for amplification/copying. However, such processing may take in the order of several hours, and is qualitative not quantitative in that it only establishes the presence of a certain DNA in the PCR reaction mixture 101, not how much of the certain DNA was present.

One type of probe that may be used in an embodiment of the present invention is SYBR® Green. SYBR® Green binds to all double-stranded DNA molecules contained in the the real-time PCR reaction mixture 151 and emits a fluorescent signal of a defined wavelength on binding. The excitation and emission maxima of SYBR^{®} Green I are at 494 nm and 521 nm, respectively. Signal intensity of the fluorescence increases with increasing cycle number due to the accumulation of PCR product. Use of fluorescent dyes may enable analysis of many different targets without having to synthesize target-specific labeled probes. However, nonspecific PCR products and primer-dimers will also contribute to the fluorescent signal. Therefore, high PCR specificity may be required when using SYBR^{®} Green I.

Another type of probe are known as QuantiProbes, which are sequence-specific fluorescently labeled probes with a fluorophore at the 3' end, and a nonfluorescent quencher and minor groove binder at the 5' end. QuantiProbes form a random structure in solution, which facilitates provides for quenching of the fluorescent signal associated with the probes. However, when the QuantiProbe hybridizes to its target sequence during the real-time PCR annealing step, step 180, the fluorophore and quencher separate and a fluorescent signal is generated. The fluorescent signal is directly proportional to the amount of PCR product present in the reaction at a given time point, enabling sensitive and accurate quantification of target sequences.

TaqMan® probes, are sequence-specific oligonucleotide probes carrying a fluorophore and a quencher dye. The fluorophore is attached at the 5' end of the probe and the quencher dye is located at the 3' end. During the combined annealing/extension phase of the real-time PCR, steps 170 and 180, the probe is cleaved by the 5' →3' exonuclease activity of Taq DNA polymerase, separating the fluorophore and the quencher dyes. This results in detectable fluorescence that is proportional to the amount of accumulated PCR product.

Real-time PCR may also be provided for by using fluorescence resonance energy transfer (FRET) probes the real-time PCR reaction mixture 151, such as LightCycler® hybridization probes. Such probes use two labeled oligonucleotide probes that bind to the PCR product in a head-to-tail fashion. When the two probes bind, their fluorophores come into close proximity, allowing energy transfer from a donor to an acceptor fluorophore. Therefore, fluorescence is detected during the annealing phase of the real-time PCR process and is proportional to the amount of PCR product.

Molecular Beacons are dual-labeled probes with a fluorophore attached at the 5' end and a quencher dye attached at the 3' end. The probes are designed so that the ends are complementary. When the probe is in solution, the two ends of the probe hybridize and form a stem-loop structure with the fluorophore and quencher in close proximity to efficiently quench the fluorescent signal. When the probe binds to the target DNA sequence, however, the stem opens and the fluorophore and the quencher separate. This separation 1 in the annealing step causes the generation of a fluorescent signal that is proportional to the amount of PCR product present.

Using the probes, real-time PCR may provide for determining the actual amount of PCR product present at a given cycle in the real-time PCR process, where the amount of PCR product may be indicated by the intensity of fluorescence. The fluorescence generated by SYBR^{®} Green I or fluorescently labeled probes may be indicative of the amount of PCR product, amplicon, in the reaction, including during the exponential phase (log-linear phase) in which the PCR product is being rapidly amplified. Outside of this exponential phase (log-linear phase), the amount of amplicon present may be a constant. By selecting the threshold within the exponential phase (log-linear phase) for all samples, it is possible to calculate the actual amount of initial starting molecules since the fluorescence intensity is directly proportional to the amount of PCR product in the exponential phase (log-linear phase) and the amount of fluorescence released during the amplification cycle is proportional to the amount of product generated in each cycle.

In embodiments of the present invention where multiple biological tags are used, the probes 173 may comprise multiple different types of probes, where each probe may be configured to produce a measurable physical property in the presence of only the PCR product associated with one type of biological tag and where the measurable physical property produced is physically distinct from that produced by a probe associated with a PCR product associated with a different biological tag. As such, physically distinct measurable physical properties are produced by each probe when in the presence of PCR product produced during amplification of the different biological tags.

As noted above, the probes are configured to provide/create/cause/be the source of a measurable physical effect that changes with the amount of PCR product. In embodiments where the measurable physical effect is fluorescence, this fluorescence may be measured during each cycle of the real-time PCR process. In this way, the dynamic range of the reaction may be greatly increased, where the dynamic range of an assay determines how much the concentration of the initial amount of biological tags in a sample may vary and still be quantified. A wide dynamic range means that a wide range of ratios of target biological tags and normaliser/reference tags can be assayed with equal sensitivity and specificity. It follows that the broader the dynamic range, the more accurate the quantitation. Furthermore, because of the specificity and complementary nature of the primers and probes, the presence or not, of several different tags can be identified from a single sample; where each probe is designed to contain a fluorphore that emits light of a different wavelength, corresponding to separate detection channels in the detection instrument.

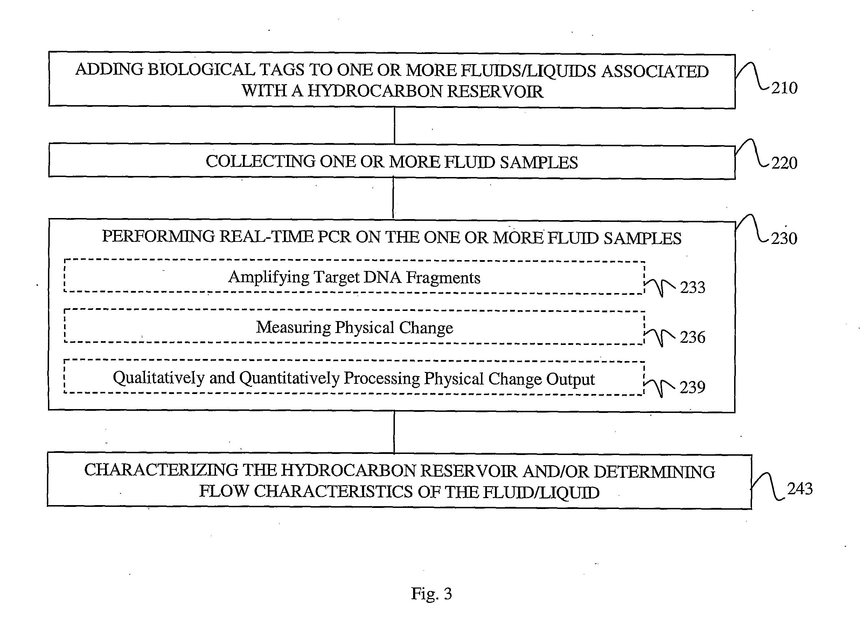

Fig. 3 is a flow type schematic illustrating a method for characterizing a hydrocarbon reservoir and/or determining flow characteristics of a liquid associated with the hydrocarbon reservoir, in accordance with an embodiment of the present invention. In step 210, a biological tag is added to a fluid associated with a hydrocarbon reservoir. Such a step may comprise adding the biological tag to a fluid being injected into the hydrocarbon reservoir, adding the biological tag to a fluid present in the hydrocarbon reservoir and/or the like. The fluid being added to and/or injected into the hydrocarbon formation may be a treatment fluid - *i.e.,* a fluid configured to provide for such things as stimulation, isolation or control of reservoir gas or water - a fluid for use in a hydraulic fracturing process - such as a pre-pad fluid, a pad fluid, a fracturing fluid, a tailing fluid or the like - and/or the like. These tags may be used to monitor wellbore fluids such as fracturing fluids, injection water, drilling fluids and other water-based treatment fluids and may also be used to determine the results of adding such fluids to the hydrocarbon reservoir or an earth formation associated with the reservoir.

Alternatively or in combination, the biological tag may be added to a fluid already in or flowing through the hydrocarbon reservoir to determine flow properties for the fluid, determine flow changes during extraction of hydrocarbons from the reservoir and/or the like. In certain aspects, different biological tags may be added to the fluids at different locations.

In step 220, a fluid sample is collected. The fluid sample may be collected from the hydrocarbon reservoir, a wellbore penetrating the hydrocarbon reservoir and/or from a formation outside of the hydrocarbon reservoir. In certain aspects, a tool may be positioned in the wellbore to collect the fluid sample. In other aspects, a probe may be extended into a formation or the hydrocarbon reservoir to collect the sample. In yet other aspects, the sample may be collected at an earth surface or from an earth formation associated with the hydrocarbon reservoir. In one embodiment of the present invention, multiple samples may be collected from one or more locations - either geographical or temporal locations - for analysis.

Once collected, the samples may be centrifuged and the collected material dispersed into buffered distilled water. In step 233, any DNA fragments in the collected samples that match the biological tags added to the liquid associated with the hydrocarbon reservoir may be amplified by mixing the collected material and buffered distilled water with nucleic acid bases, polymerase enzyme, the predetermined primers and magnesium chloride; wherein the predetermined primers are chosen to provide for DNA fragment specific amplification. By adding probes, as explained in more detail above, that are responsive to the amplification of the DNA fragments, a measurable physical property may be caused that changes in relation to the amount of the amplified DNA fragments in the mixture. As noted previously, a plurality of probes may be provided in the mixture where each probe generates a distinct measurable physical property in response to the amplification of a specific DNA fragment. Merely by way of example, where the measurable physical property is fluorescence, one type of probe may give rise fluorescence of a particular wavelength in response to amplification/presence of a first DNA fragment and a second type of probe may give rise to fluorescence of a different wavelength in response to amplification presence of a second DNA fragment.

In step 236, the measurable physical property may be measured. Merely by way of example, where the measurable physical property is fluorescence measurements may be performed using a laser and a charge-coupled device (CCD) optics system. In such a configuration, output from the laser may be passed through the real-time PCR mixture. An optical fiber may be coupled with the laser to provide for delivery of the laser output through the real-time PCR mixture via a lens. The laser light passing through the real-time PCR mixture may excite the fluorochrome in the PCR solution and the resulting emissions from the real-time PCR mixture may be detected by a CCD and analyzed. Image processing methods, such as software algorithms or the like, may then be used to identify the wavelength being detected and/or an intensity value for the wavelength.

In step 239, the measurements of the measurable physical property may be processed by a processor, software and/or the like to determine whether one or more of the biological tags is present in the sample and/or a quantitative amount of one or more of the biological tags in the sample. The output of the processor may then be further processed in step 243 and may be combined with measurements from samples taken from different locations or the same location at different time to provide for characterization of the hydrocarbon reservoir, determination of the flow characteristics of one or more fluids associated with the hydrocarbon reservoir, determinations regarding interactions of injected fluids with the hydrocarbon reservoir or surrounding earth formations and/or the like.

In a fracturing procedure carried out on the hydrocarbon reservoir a series of fluids may be injected into the hydrocarbon reservoir to achieve the fracturing effect. In an embodiment of the present invention, in such a procedure, a different DNA tag may be provided in each of the different fluids during the fracturing procedure. After the procedure is completed and production of hydrocarbons from the hydrocarbon reservoir is resumed, the fracturing fluids may be returned to the surface. In an embodiment of the present invention, samples of these fluids may be taken and submitted to real-time PCR, as described above. As such, the samples may be centrifuged and the material collected dispersed into buffered distilled water. To this mix may be added nucleic acid bases, polymerase enzyme, PCT primers for one or more of the different DNA tags, probes and magnesium chloride. The mixture may then be placed into a real-time PCR system, such as the Cepheid Smartcycler, and the amplification and detection process is performed. The data generated may provide for an indication as to which tags are in which returned fluids, the amount of tag present and how much mixing of the fluids has occurred. From this information, determinations may be made about the results of the fracturing process and/or whether clean up *etc.* may be required.

While the principles of the invention have been described above in connection with specific apparatuses and methods, it is to be clearly understood that this description is made only by way of example and not as limitation on the scope of the invention. Moreover, except where clearly inappropriate or otherwise expressly noted, it should be assumed that the features, devices and/or components of different embodiments can be substituted and/or combined. Thus, the above description should not be taken as limiting the scope of the invention, which is defined by the appended claims.

## Claims

1. A method for characterizing a hydrocarbon reservoir (25) or determining flow properties of fluids associated with a hydrocarbon reservoir (25), comprising:
adding a first biological tag to a first fluid associated with the hydrocarbon reservoir (25); and
collecting a first fluid sample from the hydrocarbon reservoir (25) or a wellbore (20) penetrating the hydrocarbon reservoir (25), **characterized by**
performing real-time PCR on the first fluid sample, which step includes attaching a first pair of complementary primers (121, 171) to the first biological tag for selective replication of the first biological tag, detecting an amount of PCR product produced by the replication of the first biological tag during one or more cycles of a PCR reaction, and using the amount of the PCR product to measure an amount of the first biological tag in the first fluid sample; and
using the measured amount of the first biological tag in the first fluid sample as determined by the real-time PCR to provide for at least one of characterizing the hydrocarbon reservoir (25) and determining the flow properties of the first fluid.

2. The method of claim 1, further comprising:
injecting a second liquid containing second biological tags into the hydrocarbon reservoir (25); and
wherein performing real-time PCR on the first fluid sample further comprises:
using a second pair of complementary primers to provide for amplification of any of the second biological tags present in the first fluid sample, wherein the second primer is configured to selectively attach to the second biological tags
using a first probe (173) to selectively detect the presence of the first biological tags or copies of the first biological tags, wherein the first probe (173) is configured to produce a first measurable physical property that varies in accordance with a first amount of the first biological tags or the copies of the first biological tags;
using a second probe to selectively detect the presence of the second biological tags, wherein the second probe is configured to produce a second measurable physical property that is distinct from the first measurable physical property and that varies in accordance with a second amount of the second biological tags or copies of the second biological tags; and
using first results from the real-time PCR to characterize the hydrocarbon reservoir (25) or determine the flow properties of the first or the second fluids in the hydrocarbon reservoir (25).

3. The method of claim 1, further comprising:
collecting a second fluid sample from the hydrocarbon reservoir (25) or a wellbore (20) penetrating the hydrocarbon reservoir (25);
performing real-time PCR on the second fluid sample to detect a presence of or measure an amount of the first biological tag in the second fluid sample; and
obtaining second results from the step of performing real-time PCR on the second fluid sample.

4. The method of claim 3 wherein the second fluid sample is collected at a location that is either geographically or temporarily distinct from where the first fluid sample is collected.

5. The method of claim 2 or claim 3, wherein the using the measured amounts of the first biological tag in the first and second fluid samples as determined by the real-time PCR are used to map flow of the first fluid in the hydrocarbon reservoir (25).

6. The method of any one of the preceding claims, wherein the step of adding a first biological tag to a first fluid associated with the hydrocarbon reservoir (25) comprises adding the first biological tag to the first fluid and injecting the first fluid into the hydrocarbon reservoir (25).

7. The method of claim 6, wherein the first fluid is injected into the hydrocarbon reservoir (25) through a coiled-tubing borehole.

8. The method of any one of the preceding claims wherein the step of performing real-time PCR on the first fluid sample to detect a presence of or measure an amount of the first biological tag in the first fluid sample comprises including an intercalating agent in a PCR reaction, the intercalating agent configured to have a detectable physical property that varies in accordance with amount of PCR product produced in the PCR reaction and measuring a value of the detectable physical property.

9. The method of any one of the preceding claims wherein the step of performing real-time PCR on the first fluid sample to measure an amount of the first biological tag in the first fluid sample comprises using a probe in a PCR reaction and measuring a detectable physical property produced by the probe, wherein the probe is configured to produce the detectable physical property in relation to an amount of PCR product produced in the PCR reaction.

10. The method of claim 9, wherein the probe is selected from a group consisting of a hydrolysis probe, a hybridising probe and a DNA-binding agent.

11. A system for characterizing a hydrocarbon reservoir (25) or determining flow properties of fluids injected into the hydrocarbon reservoir (25), comprising:
means for injecting a first liquid containing a first biological tag into the hydrocarbon reservoir (25); and
means for collecting a first fluid sample from the hydrocarbon reservoir (25) or a wellbore (20) penetrating the hydrocarbon reservoir (25), **characterized by**
means for performing real-time PCR on the fluid sample using a first pair of complementary primers (121, 171), wherein each of the first pair of complementary primers (121, 171) is configured to attach to the first biological tag and provide for selective replication of the first biological tag during the real-time PCR so as to detect an amount of PCR product produced by the replication of the first biological tag during one or more cycles of a PCR reaction and use the amount of the PCR product to measure an amount of the first biological tag in the fluid sample; and
means for using the measured amount of the first biological tag in the first fluid sample as determined by the real-time PCR to provide for at least one of characterizing the hydrocarbon reservoir (25) and determining the flow properties of the first fluid.

12. A system according to claim 11 also comprising
means for injecting a second liquid containing a second biological tag into the hydrocarbon reservoir (25);
means for performing real-time PCR on the fluid sample using a second pair of complementary primers, wherein each of the second pair of complementary primers is configured to attach to the second biological tag and provide for selective replication of the second biological tag during the real-time PCR so as to detect an amount of PCR product produced by the replication of the second biological tag during one or more cycles of a PCR reaction and use the amount of the PCR product to measure an amount of the second biological tag in the fluid sample;
means for using a first probe (173) to selectively detect the presence of the first biological tags or copies of the first biological tags, wherein the first probe (173) is configured to produce a first measurable physical property that varies in accordance with a first amount of the first biological tags or the copies of the first biological tags; and
means for using a second probe to selectively detect the presence of the second biological tags, wherein the second probe is configured to produce a second measurable physical property that is distinct from the first measurable physical property and that varies in accordance with a second amount of the second biological tags or copies of the second biological tags.

13. A system according to claim 11 also comprising means for collecting a second fluid sample from the hydrocarbon reservoir (25) or a wellbore (20) penetrating the hydrocarbon reservoir (25) and means for performing real-time PCR on the second fluid sample

14. A system according to claim 11, claim 12 or claim 13 comprising
a first well-tool configured for suspension in a wellbore (20) penetrating the hydrocarbon reservoir (25);
a pump coupled with the first well-tool and configured to pump a first liquid containing a biological tag into the hydrocarbon reservoir (25); and
a sampling chamber configured to collect a fluid sample from the hydrocarbon reservoir (25).

15. A system according to claim 14, wherein the sampling chamber is coupled with a second well-tool and suspended in the wellbore (20) to provide for collection of the fluid sample.

## Patentansprüche

1. Verfahren zum Charakterisieren eines Kohlenwasserstoff-Reservoirs (25) oder Bestimmen von Fließeigenschaften von mit einem Kohlenwasserstoff-Reservoir (25) assoziierten Flüssigkeiten, umfassend:
Hinzufügen einer ersten biologischen Markierung zu einer ersten Flüssigkeit, die mit dem Kohlenwasserstoff-Reservoir (25) assoziiert wird; und
Entnehmen einer ersten Flüssigkeitsprobe aus dem Kohlenwasserstoff-Reservoir (25) oder einem Bohrloch (20), das das Kohlenwasserstoff-Reservoir (25) penetriert, **gekennzeichnet durch**
Durchführen von Echtzeit-PCR an der ersten Flüssigkeitsprobe, wobei dieser Schritt Folgendes beinhaltet: Anhängen eines ersten Paares an komplementären Primern (121, 171) an die erste biologische Markierung zur selektiven Replikation der ersten biologischen Markierung, Erkennen einer Menge eines **durch** die Replikation der ersten biologischen Markierung während eines oder mehreren Zyklen einer PCR-Reaktion produzierten PCR-Produkts, und Verwenden der Menge des PCR-Produkts zum Messen einer Menge der ersten biologischen Markierung in der ersten Flüssigkeitsprobe; und
Verwenden der gemessenen Menge der ersten biologischen Markierung in der ersten Flüssigkeitsprobe wie **durch** die Echtzeit-PCR festgestellt, um für mindestens eines von Charakterisieren des Kohlenwasserstoff-Reservoirs (25) und Bestimmen der Fließeigenschaften der ersten Flüssigkeit zu sorgen.

2. Verfahren nach Anspruch 1, weiter umfassend:
Injizieren einer zweiten Flüssigkeit, die zweite biologische Markierungen enthält, in das Kohlenwasserstoff-Reservoir (25); und
wobei das Durchführen einer Echtzeit-PCR an der ersten Flüssigkeitsprobe weiter Folgendes umfasst:
Verwenden eines zweiten Paares an komplementären Primern, um für die Verstärkung der in der ersten Flüssigkeitsprobe vorhandenen zweiten biologischen Markierungen zu sorgen, wobei der zweite Primer so gestaltet ist, dass er sich selektiv an die zweiten biologischen Markierungen anhängt;
Verwenden einer ersten Sonde (173) zum selektiven Feststellen des Vorhandenseins der ersten biologischen Markierungen oder von Kopien der ersten biologischen Markierungen, wobei die erste Sonde (173) so gestaltet ist, dass sie eine erste messbare physikalische Eigenschaft produziert, die in Übereinstimmung mit einer ersten Menge der ersten biologischen Markierungen oder der Kopien der ersten biologischen Markierungen variiert;
Verwenden einer zweiten Sonde zum selektiven Feststellen des Vorhandenseins der zweiten biologischen Markierungen, wobei die zweite Sonde so gestaltet ist, dass sie eine zweite messbare physikalische Eigenschaft produziert, die sich von der ersten messbaren physikalischen Eigenschaft unterscheidet und die in Übereinstimmung mit einer zweiten Menge der zweiten biologischen Markierungen oder Kopien der zweiten biologischen Markierungen variiert; und
Verwenden erster Ergebnisse der Echtzeit-PCR zum Charakterisieren des Kohlenwasserstoff-Reservoirs (25) oder Bestimmen der Fließeigenschaften der ersten oder der zweiten Flüssigkeit im Kohlenwasserstoff-Reservoir (25).

3. Verfahren nach Anspruch 1, weiter umfassend:
Entnehmen einer zweiten Flüssigkeitsprobe aus dem Kohlenwasserstoff-Reservoir (25) oder einem Bohrloch (20), das das Kohlenwasserstoff-Reservoir (25) penetriert;
Durchführen von Echtzeit-PCR an der zweiten Flüssigkeitsprobe zum Feststellen eines Vorhandenseins oder Messen einer Menge der ersten biologischen Markierung in der zweiten Flüssigkeitsprobe; und
Erhalten zweiter Ergebnisse durch den Schritt des Durchführens der Echtzeit-PCR an der zweiten Flüssigkeitsprobe.

4. Verfahren nach Anspruch 3, wobei die zweite Flüssigkeitsprobe an einem Ort entnommen wird, der sich entweder geographisch oder zeitlich von dem Ort unterscheidet, an dem die erste Flüssigkeitsprobe entnommen wird.

5. Verfahren nach Anspruch 2 oder Anspruch 3, wobei das Verwenden der gemessenen Mengen der ersten biologischen Markierung in der ersten und zweiten Flüssigkeitsprobe wie durch die Echtzeit-PCR festgelegt verwendet wird, um den Fluss der ersten Flüssigkeit im Kohlenwasserstoff-Reservoir (25) abzubilden.

6. Verfahren nach einem der vorhergehenden Ansprüche, wobei der Schritt des Hinzufügens einer ersten biologischen Markierung zu einer mit dem Kohlenwasserstoff-Reservoir (25) assoziierten ersten Flüssigkeit das Hinzufügen der ersten biologischen Markierung zur ersten Flüssigkeit und Injizieren der ersten Flüssigkeit in das Kohlenwasserstoff-Reservoir (25) umfasst.

7. Verfahren nach Anspruch 6, wobei die erste Flüssigkeit durch ein Coiled-Tubing-Bohrloch in das Kohlenwasserstoff-Reservoir (25) injiziert wird.

8. Verfahren nach einem der vorhergehenden Ansprüche, wobei der Schritt des Durchführens der Echtzeit-PCR an der ersten Flüssigkeitsprobe zum Feststellen eines Vorhandenseins von oder Messen einer Menge der ersten biologischen Markierung in der ersten Flüssigkeitsprobe das Einbeziehen eines interkalierenden Stoffes in eine PCR-Reaktion, wobei der interkalierende Stoff so gestaltet ist, dass er eine feststellbare physikalische Eigenschaft hat, die in Übereinstimmung mit der Menge des in der PCR-Reaktion produzierten PCR-Produkts variiert, und das Messen eines Wertes der feststellbaren physikalischen Eigenschaft umfasst.

9. Verfahren nach einem der vorhergehenden Ansprüche, wobei der Schritt des Durchführens der Echtzeit-PCR an der ersten Flüssigkeitsprobe zum Messen einer Menge der ersten biologischen Markierung in der ersten Flüssigkeitsprobe das Verwenden einer Sonde in einer PCR-Reaktion und Messen einer durch die Sonde produzierten feststellbaren physikalischen Eigenschaft umfasst, wobei die Sonde so gestaltet ist, dass sie die feststellbare physikalische Eigenschaft in Bezug auf eine Menge eines in der PCR-Reaktion produzierten PCR-Produkts produziert.

10. Verfahren nach Anspruch 9, wobei die Sonde aus einer Gruppe gewählt wird, die aus einer Hydrolysesonde, einer Hybridisierungssonde und einem DNA-bindenden Stoff besteht.

11. System zum Charakterisieren eines Kohlenwasserstoff-Reservoirs (25) oder Bestimmen von Fließeigenschaften von in das Kohlenwasserstoff-Reservoir (25) injizierten Flüssigkeiten, umfassend:
Mittel zum Injizieren einer ersten Flüssigkeit, die eine erste biologische Markierung enthält, in das Kohlenwasserstoff-Reservoir (25); und
Mittel zum Entnehmen einer ersten Flüssigkeitsprobe aus dem Kohlenwasserstoff-Reservoir (25) oder einem Bohrloch (20), das das Kohlenwasserstoff-Reservoir (25) penetriert, **gekennzeichnet durch**
Mittel zum Durchführen von Echtzeit-PCR an der Flüssigkeitsprobe unter Verwendung eines ersten Paares an komplementären Primern (121, 171), wobei jeder des ersten Paares an komplementären Primern (121, 171) so gestaltet ist, dass er sich an die erste biologische Markierung anhängt und für die selektive Replikation der ersten biologischen Markierung während der Echtzeit-PCR sorgt, um eine Menge eines **durch** die Replikation der ersten biologischen Markierung während eines oder mehrerer Zyklen einer PCR-Reaktion produzierten PCR-Produkts zu erkennen und die Menge des PCR-Produkts zu verwenden, um eine Menge der ersten biologischen Markierung in der Flüssigkeitsprobe zu messen; und
Mittel zum Verwenden der gemessenen Menge der ersten biologischen Markierung in der ersten Flüssigkeitsprobe wie **durch** die Echtzeit-PCR festgestellt, um für mindestens eines des Charakterisierens des Kohlenwasserstoff-Reservoirs (25) und Bestimmen der Fließeigenschaften der ersten Flüssigkeit zu sorgen.

12. System nach Anspruch 11, weiter umfassend
Mittel zum Injizieren einer zweiten Flüssigkeit, die eine zweite biologische Markierung enthält, in das Kohlenwasserstoff-Reservoir (25);
Mittel zum Durchführen von Echtzeit-PCR an der Flüssigkeitsprobe unter Verwendung eines zweiten Paares an komplementären Primern, wobei jeder des zweiten Paares an komplementären Primern so gestaltet ist, dass er sich an die zweite biologische Markierung anhängt und für die selektive Replikation der zweiten biologischen Markierung während der Echtzeit-PCR sorgt, um eine Menge eines durch die Replikation der zweiten biologischen Markierung während eines oder mehrerer Zyklen einer PCR-Reaktion produzierten PCR-Produkts zu erkennen und die Menge des PCR-Produkts zu verwenden, um eine Menge der zweiten biologischen Markierung in der Flüssigkeitsprobe zu messen;
Mittel zum Verwenden einer ersten Sonde (173) zum selektiven Feststellen des Vorhandenseins der ersten biologischen Markierungen oder Kopien der ersten biologischen Markierungen, wobei die erste Sonde (173) so gestaltet ist, dass sie eine erste messbare physikalische Eigenschaft produziert, die in Übereinstimmung mit einer ersten Menge der ersten biologischen Markierungen oder der Kopien der ersten biologischen Markierungen variiert; und
Mittel zum Verwenden einer zweiten Sonde zum selektiven Feststellen des Vorhandenseins der zweiten biologischen Markierungen, wobei die zweite Sonde so gestaltet ist, dass sie eine zweite messbare physikalische Eigenschaft produziert, die sich von der ersten messbaren physikalischen Eigenschaft unterscheidet und die in Übereinstimmung mit einer zweiten Menge der zweiten biologischen Markierungen oder Kopien der zweiten biologischen Markierungen variiert.

13. System nach Anspruch 11, auch umfassend Mittel zum Entnehmen einer zweiten Flüssigkeitsprobe aus dem Kohlenwasserstoff-Reservoir (25) oder einem Bohrloch (20), das das Kohlenwasserstoff-Reservoir (25) penetriert und Mittel zum Durchführen von Echtzeit-PCR an der zweiten Flüssigkeitsprobe.

14. System nach Anspruch 11, Anspruch 12 oder Anspruch 13, umfassend ein erstes Bohrwerkzeug, das zur Suspension in einem Bohrloch (20), das das Kohlenwasserstoff-Reservoir (25) penetriert, gestaltet ist;
eine mit dem ersten Bohrwerkzeug gekoppelte Pumpe, die so gestaltet ist, dass sie eine erste Flüssigkeit, die eine erste biologische Markierung enthält, in das Kohlenwasserstoff-Reservoir (25) pumpt; und
eine Probenahmekammer, die so konfiguriert ist, dass sie dem Kohlenwasserstoff-Reservoir (25) eine Flüssigkeitsprobe entnimmt.

15. System nach Anspruch 14, wobei die Probenahmekammer mit einem zweiten Bohrwerkzeug gekoppelt und im Bohrloch (20) suspendiert ist, um für die Entnahme der Flüssigkeitsprobe zu sorgen.

## Revendications

1. Procédé pour caractériser un réservoir d'hydrocarbure (25) ou déterminer des propriétés rhéologiques de fluides associés à un réservoir d'hydrocarbure (25), comprenant :
l'ajout d'un premier marqueur biologique à un premier fluide associé au réservoir d'hydrocarbure (25) ; et
le recueil d'un premier échantillon de fluide du réservoir d'hydrocarbure (25) ou d'un trou de forage (20) pénétrant le réservoir d'hydrocarbure (25), **caractérisé par** les étapes consistant à
exécuter une PCR en temps réel sur le premier échantillon de fluide, laquelle étape comprend l'attachement d'une première paire d'amorces complémentaires (121, 171) au premier marqueur biologique pour la réplication sélective du premier marqueur biologique, la détection d'une quantité de produit PCR produite par la réplication du premier marqueur biologique pendant un ou plusieurs cycles d'une réaction PCR, et l'utilisation de la quantité du produit PCR pour mesurer une quantité du premier marqueur biologique dans le premier échantillon de fluide ; et
utiliser la quantité mesurée du premier marqueur biologique dans le premier échantillon de fluide telle que déterminée par la PCR en temps réel pour assurer au moins une parmi la caractérisation du réservoir d'hydrocarbure (25) et la détermination des propriétés rhéologiques du premier fluide.

2. Procédé selon la revendication 1, comprenant en outre :
l'injection d'un second liquide contenant des seconds marqueurs biologiques dans le réservoir d'hydrocarbure (25) ; et
dans lequel l'exécution de la PCR en temps réel sur le premier échantillon de fluide comprend en outre :
l'utilisation d'une seconde paire d'amorces complémentaires pour assurer l'amplification de l'un quelconque des seconds marqueurs biologiques présents dans le premier échantillon de fluide, la seconde amorce étant configurée pour s'attacher sélectivement aux seconds marqueurs biologiques ;
l'utilisation d'une première sonde (173) pour détecter sélectivement la présence des premiers marqueurs biologiques ou de copies des premiers marqueurs biologiques, la première sonde (173) étant configurée pour produire une première propriété physique mesurable qui varie en fonction d'une première quantité des premiers marqueurs biologiques ou des copies des premiers marqueurs biologiques ;
l'utilisation d'une seconde sonde pour détecter sélectivement la présence des seconds marqueurs biologiques, la seconde sonde étant configurée pour produire une seconde propriété physique mesurable qui est distincte de la première propriété physique mesurable et qui varie en fonction d'une seconde quantité des seconds marqueurs biologiques ou de copies des seconds marqueurs biologiques ; et
l'utilisation de premiers résultats issus de la PCR en temps réel pour caractériser le réservoir d'hydrocarbure (25) ou déterminer les propriétés rhéologiques des premier ou second fluides dans le réservoir d'hydrocarbure (25).

3. Procédé selon la revendication 1, comprenant en outre :
le recueil d'un second échantillon de fluide du réservoir d'hydrocarbure (25) ou d'un trou de forage (20) pénétrant le réservoir d'hydrocarbure (25) ;
l'exécution d'une PCR en temps réel sur le second échantillon de fluide pour détecter une présence de ou mesurer une quantité du premier marqueur biologique dans le second échantillon de fluide ; et
l'obtention de seconds résultats issus de l'étape d'exécution de la PCR en temps réel sur le second échantillon de fluide.

4. Procédé selon la revendication 3, dans lequel le second échantillon de fluide est recueilli en un emplacement qui est soit géographiquement, soit temporairement distinct de l'endroit où le premier échantillon de fluide est recueilli.

5. Procédé selon la revendication 2 ou 3, dans lequel les quantités mesurées du premier marqueur biologique dans les premier et second échantillons de fluide telles que déterminées par la PCR en temps réel sont utilisées pour cartographier l'écoulement du premier fluide dans le réservoir d'hydrocarbure (25).

6. Procédé selon l'une quelconque des revendications précédentes, dans lequel l'étape consistant à ajouter un premier marqueur biologique à un premier fluide associé au réservoir d'hydrocarbure (25) comprend l'ajout du premier marqueur biologique au premier fluide et l'injection du premier fluide dans le réservoir d'hydrocarbure (25).

7. Procédé selon la revendication 6, dans lequel le premier fluide est injecté dans le réservoir d'hydrocarbure (25) par un trou de sondage à tube spiralé.

8. Procédé selon l'une quelconque des revendications précédentes, dans lequel l'étape d'exécution d'une PCR en temps réel sur le premier échantillon de fluide pour détecter une présence de ou mesurer une quantité du premier marqueur biologique dans le premier échantillon de fluide comprend l'inclusion d'un agent intercalant dans une réaction PCR, l'agent intercalant étant configuré pour avoir une propriété physique détectable qui varie en fonction de la quantité de produit PCR produite dans la réaction PCR, et la mesure d'une valeur de la propriété physique détectable.

9. Procédé selon l'une quelconque des revendications précédentes, dans lequel l'étape d'exécution de la PCR en temps réel sur le premier échantillon de fluide pour mesurer une quantité du premier marqueur biologique dans le premier échantillon de fluide comprend l'utilisation d'une sonde dans une réaction PCR et la mesure d'une propriété physique détectable produite par la sonde, la sonde étant configurée pour produire la propriété physique détectable en rapport avec une quantité de produit PCR produite dans la réaction PCR.

10. Procédé selon la revendication 9, dans lequel la sonde est sélectionnée parmi un groupe comprenant une sonde d'hydrolyse, une sonde d'hybridation et un agent de liaison à l'ADN.

11. Système pour caractériser un réservoir d'hydrocarbure (25) ou déterminer les propriétés rhéologiques de fluides injectés dans le réservoir d'hydrocarbure (25), comprenant :
des moyens pour injecter un premier liquide contenant un premier marqueur biologique dans le réservoir d'hydrocarbure (25) ; et
des moyens pour recueillir un premier échantillon de fluide du réservoir d'hydrocarbure (25) ou d'un trou de forage (20) pénétrant le réservoir d'hydrocarbure (25), **caractérisé par**
des moyens pour exécuter une PCR en temps réel sur l'échantillon de fluide au moyen d'une première paire d'amorces complémentaires (121, 171), chacune de la première paire d'amorces complémentaires (121, 171) étant configurée pour s'attacher au premier marqueur biologique et assurer une réplication sélective du premier marqueur biologique pendant la PCR en temps réel de manière à détecter une quantité de produit PCR produite par la réplication du premier marqueur biologique pendant un ou plusieurs cycles d'une réaction PCR, et utiliser la quantité du produit PCR pour mesurer une quantité du premier marqueur biologique dans l'échantillon de fluide ; et
des moyens pour utiliser la quantité mesurée du premier marqueur biologique dans le premier échantillon de fluide telle que déterminée par la PCR en temps réel pour assurer au moins une parmi la caractérisation du réservoir d'hydrocarbure (25) et la détermination des propriétés rhéologiques du premier fluide.

12. Système selon la revendication 11, comprenant également :
des moyens pour injecter un second liquide contenant un second marqueur biologique dans le réservoir d'hydrocarbure (25) ;
des moyens pour exécuter une PCR en temps réel sur l'échantillon de fluide au moyen d'une seconde paire d'amorces complémentaires, chacune de la seconde paire d'amorces complémentaires étant configurée pour s'attacher au second marqueur biologique et assurer la réplication sélective du second marqueur biologique pendant la PCR en temps réel de manière à détecter une quantité de produit PCR produite par la réplication du second marqueur biologique pendant un ou plusieurs cycles d'une réaction PCR et utiliser la quantité du produit PCR pour mesurer une quantité du second marqueur biologique dans l'échantillon de fluide ;
des moyens pour utiliser une première sonde (173) pour détecter sélectivement la présence des premiers marqueurs biologiques ou de copies des premiers marqueurs biologiques, la première sonde (173) étant configurée pour produire une première propriété physique mesurable qui varie en fonction d'une première quantité des premiers marqueurs biologiques ou des copies des premiers marqueurs biologiques ; et
des moyens pour utiliser une seconde sonde pour détecter sélectivement la présence des seconds marqueurs biologiques, la seconde sonde étant configurée pour produire une seconde propriété physique mesurable qui est distincte de la première propriété physique mesurable et qui varie en fonction d'une seconde quantité des seconds marqueurs biologiques ou de copies des seconds marqueurs biologiques.

13. Système selon la revendication 11, comprenant également des moyens pour recueillir un second échantillon de fluide du réservoir d'hydrocarbure (25) ou d'un trou de forage (20) pénétrant le réservoir d'hydrocarbure (25) et des moyens pour exécuter une PCR en temps réel sur le second échantillon de fluide.

14. Système selon la revendication 11, 12 ou 13 comprenant :
un premier outil de forage configuré pour être suspendu dans un trou de forage (20) pénétrant le réservoir d'hydrocarbure (25) ;
une pompe couplée au premier outil de forage et configurée pour injecter par pompage un premier liquide contenant un marqueur biologique dans le réservoir d'hydrocarbure (25) ; et
une chambre d'échantillonnage configurée pour recueillir un échantillon de fluide du réservoir d'hydrocarbure (25).

15. Système selon la revendication 14, dans lequel la chambre d'échantillonnage est couplée à un second outil de forage et suspendue dans le trou de forage (20) pour assurer le recueil de l'échantillon de fluide.
